# FASCICULE DE BREVET EUROPEEN

(11) **EP 0 431 108 B1**
(45) Date de publication et mention de la délivrance du brevet: **08.12.1993**
(21) Numéro de dépôt: 90908562.3
(22) Date de dépôt: 01.06.1990
(51) Int. Cl.: A61M 16/04, A61M 16/20

(54) **DISPOSITIF D'OXYGENO-PHONATION**
SPRECHEINRICHTUNG ZUM GEBRAUCH BEI SAUERSTOFFVERSORGUNG
OXYGENO-PHONATION DEVICE

(30) Priorité: 05.06.1989 FR 8907625
(43) Date de publication de la demande: 12.06.1991
(73) Titulaire: HUCHON, Jean-Michel, F-70190 Rioz (FR)
(72) Inventeur: Huchon, Jean-Michel, F-70190 Rioz (FR); Vannson, Philippe, F-25170 Recologne (FR)
(74) Mandataire: Ropital-Bonvarlet, Claude
(86) Numéro de dépôt international: FR9000382
(87) Numéro de publication internationale: WO9014854

(56) Documents cités:
- DE-U- 8 701 414
- US-A- 3 683 931
- US-A- 4 325 366

## Description

### DOMAINE TECHNIQUE :

La présente invention concerne les valves et dispositifs d'aide à la respiration qui sont mis en place sur des patients ayant subi une trachéotomie.

Cette opération, qui consiste à ouvrir la trachée pour rétablir la respiration, est indiquée en cas de maladie grave du larynx, tel un oedème de la glotte ou, encore, par exemple, une insuffisance respiratoire chronique grave par syndrome obstructif, restrictif ou mixte sévère.

Après l'ouverture de la trachée, on procède à la mise en place d'une canule, dénommée canule de trachéotomie, par laquelle l'air extérieur peut pénétrer, permettant ainsi d'assurer la ventilation pulmonaire et l'aspiration des muscosités bronchiques.

L'implantation d'une canule de trachéotomie dans la trachée d'un patient ne permet pas, généralement, le passage de l'air inspiré en direction des voies respiratoires supérieures qui sont chargées d'assurer le fonctionnement des cordes vocales pour permettre au sujet appareillé d'exprimer des phonémes.

### TECHNIQUE ANTERIEURE :

Pour permettre aux patients, dans la trachée desquels une canule de trachéotomie est implantée, de conserver la possibilité d'exprimer des phonèmes, il a déjà été proposé de disposer, à l'extrémité externe du canal de la canule de trachéotomie, une valve incluant un clapet anti-retour permettant au flux d'air inspiré de pénétrer dans la trachée, alors que l'air expiré est bloqué par le clapet anti-retour à l'intérieur de la trachée et est ainsi conduit et forcé vers les cordes vocales du patient, dans la mesure où le type de canule de trachéotomie l'y autorise. Ces dispositifs antérieurs sont, par exemple, illustrés par la demande **DE-U-8 701 414** qui propose de monter, à l'intérieur d'une pièce d'adaptation rapportée à l'extrémité de la canule de trachéotomie, un clapet rigide cintré précontraint élastiquement en appui contre un siège annulaire. Une telle réalisation est délicate à mettre en oeuvre, en raison, notamment, de la difficulté pour régler et maintenir le cintrage et la précontrainte du clapet à des valeurs autorisant, à la fois, une respiration et une phonation normales. Une telle réalisation ne permet pas, par ailleurs, le raccordement à une installation d'oxygénation forcée, sans enlever la pièce d'adaptation comportant le clapet.

Dans certains cas, il arrive que la fonction d'hématose de sujets trachéotomisés s'avère spontanément insuffisante, ce qui nécessite le branchement sur la canule d'une source d'oxygène additionnel assurant une oxygénation forcée.

Dans de telles situations, les dispositifs de l'art antérieur se sont avérés impropres à assurer, à la fois, la fonction de phonation et la fonction d'oxygénation forcée conduisant, en cas de raccordement de l'installation d'oxygénation, à enlever le clapet anti-retour et à le remplacer par un adaptateur. Le patient, qui est alors en état d'oxygénation forcée, ne dispose plus de l'usage de la parole, ce qui présente, bien évidemment, un désavantage certain pour le malade et s'avère, en particulier, impliquer des conséquences psychologiques particulièrement négatives.

On connaît, également, le brevet **US-A-3** **683** **931** qui décrit une valve rigide mobile axialement à l'intérieur du canal d'un adaptateur et maintenue en appui élastique par l'intermédiaire d'un ressort hélicoidal. Une telle réalisation est également délicate à mettre en oeuvre et le réglage de l'élasticité du ressort, qui conditionne la fiabilité du dispositif, s'avère difficile. Il est prévu de raccorder le dispositif à une source de fluide, du type nébulisateur, sans oxygénation forcée.

L'objet de l'invention vise à réaLiser un dispositif de phonation et d'aide à la respiration pour un patient ayant subi une trachéotomie, ne présentant pas les inconvénients des dispositifs de l'art antérieur et permettant au patient de conserver l'usage de la parole, tout en ayant la possibilité de subir une oxygénation forcée.

Un autre objet de l'invention vise à réaliser une valve d'oxygéno-phonation incluant un clapet anti-retour dont le fonctionnement est particulièrement sûr et dont le remplacement peut être effectué d'une manière simple.

Un autre objet de l'invention est de proposer un dispositif d'aide à la respiration comportant une canule de trachéotomie et une valve d'oxygéno-phonation assurant, en toute sécurité et de manière durable dans le temps, les fonctions d'oxygénation et de phonation.

### EXPOSE DE L'INVENTION :

Les buts assignés à la présente invention sont atteints grâce à un dispositif de phonation et d'aide à la respiration pour un patient trachéotomisé, dispositif du type comportant un embout de canule de trachéotomie définissant un canal, dans lequel est disposée une valve de phonation constituée par un clapet anti-retour pour empêcher le passage du flux d'air expiré et autoriser le passage du flux d'air inspiré et par un moyen d'appui pour ledit clapet, ce dernier étant monté, par un moyen de fixation définissant une zone de fixation, sur une paroi transversale appartenant au moyen d'appui et constituée d'au moins deux croisillons définissant des passages pour les flux d'air et se joignant en une zone commune formant la zone fixation et coaxiale à l'embout, ledit clapet étant disposé en aval de la paroi transversale par rapport au flux d'air aspiré et étant apte à se déformer à partir de sa zone de fixation sous l'effet du flux d'air aspiré et à laisser passer celui-ci,
caractérisé en ce que :
- le clapet coopère avec une face d'appui qui appartient au moyen d'appui et qui est constituée par l'une des faces de la paroi transversale, c'est-à-dire des deux croisillons et de la zone commune centrale formant cette paroi transversale,
- ledit clapet est, d'une part, conçu et réalisé en un matériau souple et déformable et, d'autre part, est non précontraint, de telle sorte qu'il puisse être maintenu en appui étanche contre la face d'appui sous l'effet du flux d'air expiré,
- et en ce qu'il comprend, outre la valve de phonation, un moyen de raccordement à une source de fluide à titre d'élément d'aide à la respiration, ledit moyen de raccordement étant une prise de raccordement prévue dans l'embout et destinée à un raccordement à une installation d'oxygénation forcée.

Diverses autres caractéristiques ressortent de la description faite ci-dessous en référence aux dessins annexés qui montrent, à titre d'exemples non limitatifs, des formes de réalisation de l'objet de l'invention.

### BREVE DESCRIPTION DES DESSINS :

La **fig.** **1** est une vue en perspective du dispositif conforme à l'invention.

La **fig.** **2** est une vue du dispositif selon l'invention, prise selon une coupe longitudinale.

La **fig.** **3** est une vue en coupe du dispositif conforme à l'invention, prise selon la ligne **III-III** de la **fig.** **2.**

La **fig.** **4** est une vue prise selon une coupe transversale effectuée selon la ligne **IV-IV** de la **fig.** **2.**

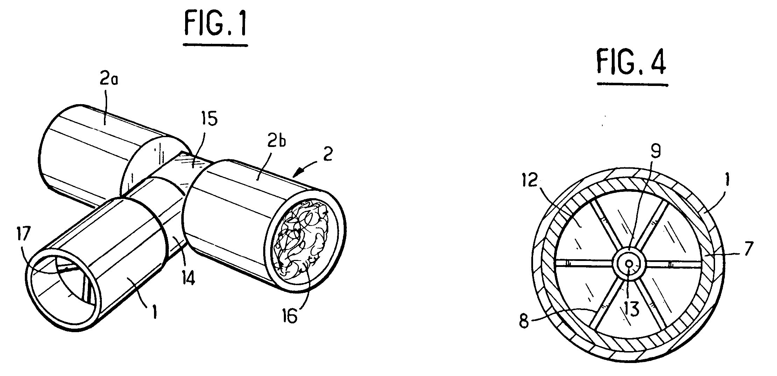

La **fig.** **1** montre une vue générale d'un dispositif d'aide à la respiration, destiné à être implanté dans la trachée d'un malade et comprenant un embout **1** sur lequel est montée une unité de filtration **2** du flux d'air.

### MEILLEURE MANIERE DE REALISER L'INVENTION :

Les exemples, représentés aux **fig.** **1 à 4**, montrent des embouts **1** de canules de trachéotomie, ainsi que des unités de filtration **2**, qui présentent des sections transversales cylindriques. Il est bien évident que les sections de ces deux éléments peuvent présenter d'autres formes géométriques et les sections transversales peuvent, en particulier, présenter des formes en carré, en losange, en hexagone ou en rectangle, sans pour autant sortir du cadre de l'invention.

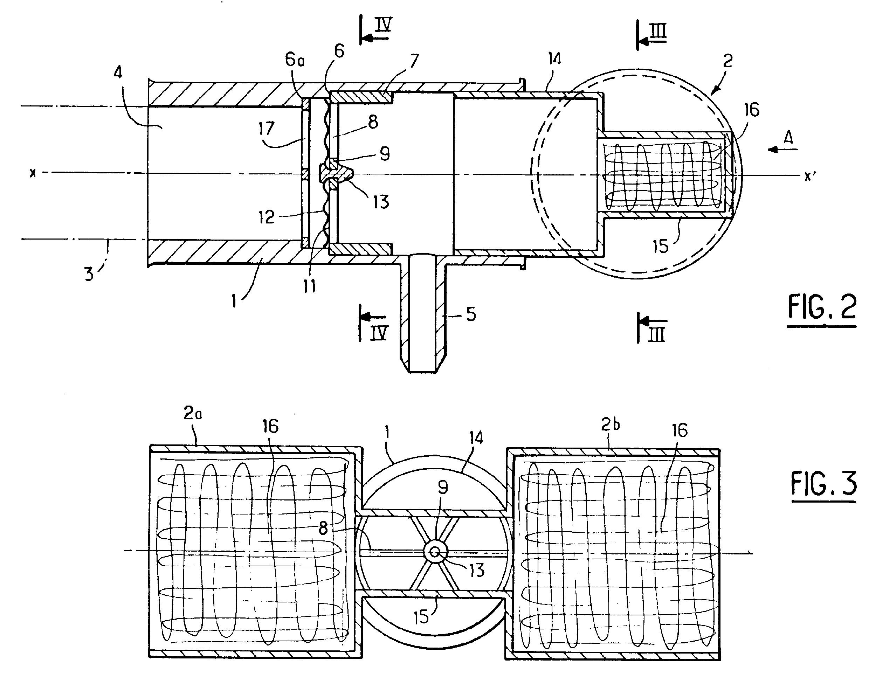

L'embout **1**, représenté à la **fig. 2**, est, généralement, implanté sur le patient par l'intermédiaire d'une canule de trachéotomie **3** dont la représentation est schématisée à la **fig.** **2.** L'embout **1** de la canule de trachéotomie **3**, qui peut être réalisé en matériau plastique par exemple, délimite un canal **4** interne dont le diamètre est, de préférence, sensiblement constant le long de l'embout **1**.

Une prise de raccordement **5**, à un dispositif d'oxygénation annexe, est prévue dans l'embout **1** et comprend un raccord tubulaire proprement dit et un perçage à travers la paroi de l'embout **1**. De préférence, le raccord tubulaire est radial et perpendiculaire à l'axe principal du canal **4**.

Le canal **4** comporte, de préférence, au moins un épaulement annulaire **6** ménagé en aval de la prise de raccordement **5** par rapport au flux d'air aspiré, dont la direction est représentée par la flèche **A**. Ces épaulements internes **6** sont utilisés comme organes de positionnement pour des éléments destinés à être mis en place dans le canal **4**.

Le dispositif d'aide à la respiration selon l'invention comprend une valve de phonation constituée d'un moyen d'appui **7** disposé dans le canal **4**. De préférence, le moyen d'appui **7** est une douille rapportée et bloquée en position dans le canal **4** de la canule contre l'épaulement annulaire **6**. La douille comporte une paroi transversale s'étendant à travers la section du canal **4**. La paroi ainsi définie est perméable au flux d'air aspiré et expiré et est, de préférence, constituée d'une série de croisillons **8** constituant des orifices de passage pour le flux d'air. Les croisillons **8** se joignent dans une zone sensiblement centrale constituée d'un anneau **9**.

L'épaisseur de la paroi transversale du moyen d'appui **7**, en l'occurence de la douille, définit une face aval **11** par rapport au flux d'air aspiré **A**, formant une face d'appui pour un clapet anti-retour **12** monté en appui étanche contre la face **11**. Le clapet anti-retour **12** est choisi avec une forme telle qu'il obstrue complètement le canal **4** et est maintenu en appui étanche, de préférence, par un clip **13** engagé à force dans la partie centrale de l'anneau **9** et bloqué élastiquement dans l'anneau **9**.

Le clapet anti-retour **12** est choisi dans un matériau souple et élastique, tel du latex siliconé ou non, susceptible de présenter des propriétés de déformation à partir de la zone de fixation définie par la liaison fixe entre le clapet anti-retour **12** et le moyen d'appui **7**, à savoir le clip **13** et l'anneau **9**. Ces propriétés de déformation incluent une déformation radiale à partir de la zone de fixation et axiale, c'est-à-dire dans le sens du flux d'air aspiré.

Il doit être considéré que la face d'appui de la douille comporte, avantageusement, au moins deux croisillons **8**, mais que d'autres moyens équivalents peuvent également être mis en oeuvre comme, par exemple, un ensemble grillagé constitué de barreaux ou, encore, un ensemble muni d'orifices de forme circulaire par exemple. IL est également envisageable de réaliser la zone d'appui et de fixation du clapet anti-retour sur la douille en une zone excentrée par rapport à l'axe **x-x'** de l'embout **1**, voire même en une zone située sur la circonférence externe de la douille. Il est, bien évidemment, également envisageable et plus particulièrement dans le dernier cas visé, de prévoir plusieurs points de fixation du clapet anti-retour **12** sur la douille **7**.

La **fig.** **2** montre, également, que le clapet anti-retour **12** est, de préférence, constitué d'une série d'ondulations aptes à lui conférer une souplesse et une élasticité accentuées.

L'extrémité opposée à la partie de l'embout **1**, qui est mise en place sur la canule de trachéotomie **3**, est destinée à recevoir une unité de filtration **2** qui, dans les exemples de représentation montrés aux **fig.** **1** et **3**, est, de préférence, constituée de deux éléments filtrants **2a** et **2b**.

L'unité de filtration **2** comporte un embout d'adaptation **14**, de section quasi identique à celle de l'embout **1**, pour pouvoir être inséré en force et de manière étanche à L'intérieur du canal **4** et y être bloqué en position au moyen, par exemple, de systèmes du type morse. L'embout d'adaptation **14** se prolonge par deux branches divergentes **2a** et **2b**, qui peuvent être de section cylindrique, d'axes longitudinaux perpendiculaires à l'axe **x'x'** de l'embout **1**, de manière à conférer à l'unité de filtration **2** la forme générale d'un **"T"**. La jonction, entre les deux branches divergentes **2a**, **2b** et l'embout **14**, est constituée d'une zone de transition **15** dont la section transversale est de dimension inférieure à la section transversale de chacune des branches divergentes **2a**, **2b**, pour constituer un **Venturi**. Avantageusement, les éléments de filtration se composent de cartouches filtrantes **16** introduites dans chacune des branches **2a**, **2b** et venant en appui contre les épaulements de chacune des branches **2a**, **2b** résultant de la réduction de section de la zone de jonction **15**. Il est, également, envisageable de prévoir un système de régulation de l'effet **Venturi** à commande manuelle.

Il est bien évident, qu'à la place d'une unité de filtration **2** pourvue de deux branches divergentes formant un **"T"**, une unité de filtration, comportant une seule branche située dans le prolongement de l'embout **1**, est également envisageable. Il est également possible de réaliser une unité de filtration comportant plus de deux cartouches de filtration **16**.

Lors de l'aspiration du flux d'air effectuée par le patient, l'air inspiré est tout d'abord filtré à l'intérieur des cartouches **16**, puis est accéléré par le système de **Venturi** formé par la réduction de section de la zone de jonction **15**, pour venir exercer une pression sur le clapet anti-retour **12** qui se déforme alors radialement et axialement dans le sens du flux d'air inspiré **A** pour laisser pénétrer le flux d'air dans le larynx du patient.

Lors de l'expiration du flux d'air, le clapet anti-retour, non précontraint, **12** est appliqué de maniére étanche contre la face d'appui de la douille et forme ainsi un obstacle au passage de l'air à travers la canule **4**, permettant alors au patient d'utiliser l'air emprisonné dans la trachée pour faire vibrer ses cordes vocales. Dans le cas où l'état du patient nécessite une oxygénation forcée, l'oxygène pénètre par la prise de raccordement **5** et déforme radialement le clapet anti-retour **12**, de la même façon que précédemment défini, puis se trouve également bloqué à l'intérieur du larynx du patient, permettant à ce dernier de conserver L'usage de la parole, même en situation d'oxygénation forcée, la prise de raccordement **5** se trouvant en amont du clapet anti-retour **12** par rapport à l'air inspiré.

Il est également envisageable de prévoir un second épaulement annulaire **6a** situé en aval de l'épaulement annulaire **6** destiné à recevoir le moyen d'appui **7**, afin de servir de siège de réception à un dispositif de sécurité constitué d'une paroi de séparation **17** du canal **4** susceptible d'empêcher le passage du clapet anti-retour **12** dans la trachée du patient. En effet, il peut advenir, qu'en raison d'une défaillance du moyen de fixation **13**, le clapet anti-retour **12** soit amené, lors de l'inspiration, à pénétrer dans la trachée. A cet effet, la paroi de séparation **17** consiste, avantageusement, en un disque rendu perméable au flux d'air, au moyen d'orifices prévus dans son épaisseur. La perméabilité de la paroi **17** peut être obtenue par une structure à croisillons, analogue à celle décrite pour la face d'appui du moyen d'appui **7** ou bien, encore, consister en une structure grillagée ou ajourée.

Le dispositif ainsi décrit permet à un sujet trachéotomisé de conserver l'usage de ses cordes vocales, même en cas d'oxygénation forcée, grâce à la valve et au dispositif d'oxygéno-phonation proposé. Le montage du clapet anti-retour **12**, sur un moyen d'appui **7** rapporté à l'intérieur du canal **4**, permet, le cas échéant, un changement aisé et rapide de la valve de phonation.

L'invention n'est pas limitée aux exemples décrits et représentés, car diverses modifications peuvent y être apportées sans sortir de son cadre et, en particulier, plusieurs clapets anti-retour peuvent être disposés dans la section du canal **4**.

### POSSIBILITE D'APPLICATION INDUSTRIELLE :

L'invention trouve une application préférée dans la réalisation d'embouts de canule de trachéotomie raccordables à des installations d'oxygénation forcée pour constituer des dispositifs d'oxygéno-phonation.

## Revendications

1. Dispositif de phonation et d'aide à la respiration pour un patient trachéotomisé, dispositif du type comportant un embout (**1**) de canule de trachéotomie (**3**) définissant un canal (**4**), dans lequel est disposée une valve de phonation constituée par un clapet anti-retour (**12**) pour empêcher le passage du flux d'air expiré et autoriser le passage du flux d'air inspiré et par un moyen d'appui (**7**) pour ledit clapet, ce dernier étant monté, par un moyen de fixation (**13**) définissant une zone de fixation (**9**), sur une paroi transversale appartenant au moyen d'appui et constituée d'au moins deux croisillons (**8**) définissant des passages pour les flux d'air et se joignant en une zone commune formant la zone fixation (**9**) coaxiale à l'embout, ledit clapet étant apte à se déformer à partir de sa zone de fixation (**9**) sous l'effet du flux d'air aspiré et à laisser passer celui-ci,
caractérisé en ce que :
- le clapet coopère avec une face d'appui (**11**) qui appartient au moyen d'appui et qui est constituée par l'une des faces de la paroi transversale, c'est-à-dire des deux croisillons et de la zone commune centrale formant cette paroi transversale,
- ledit clapet est, d'une part, conçu et réalisé en un matériau souple et déformable, et, d'autre part, est non précontraint, de telle sorte qu'il puisse être maintenu en appui étanche contre la face d'appui sous l'effet du flux d'air expiré,
et en ce qu'il comprend, outre la valve de phonation, un moyen de raccordement à une source de fluide à titre d'élément d'aide à la respiration, ledit moyen de raccordement étant une prise de raccordement (**5**) prévue dans l'embout (**1**) et destinée à un raccordement à une installation d'oxygénation forcée.

2. Dispositif selon la revendication 1, caractérisée en ce que le moyen d'appui (**7**) comporte une douille rapportée et bloquée en position dans le canal (**4**) de l'embout (**1**).

3. Dispositif selon la revendication 1 ou 2, caractérisée en ce que le moyen de fixation (**13**) est un clip de fixation enserrant le clapet anti-retour (**12**) et la zone commune (**9**) des croisillons (**8**), pour définir la zone de fixation qui est sensiblement centrale au canal (**4**) de l'embout (**1**) et à la surface délimitée par le clapet (**12**).

4. Dispositif selon la revendication 1, caractérisé en ce qu'il comprend, en outre, une paroi de séparation (**17**), perméable au flux d'air, au moyen d'orifices prévus dans son épaisseur, interposée et fixée dans le canal (**4**) de l'embout (**1**) en aval de la valve de phonation par rapport au flux d'air aspiré.

5. Dispositif d'aide à la respiration selon la revendication 4, caractérisé en ce que la paroi perméable (**17**) est constituée de croisillons.

6. Dispositif d'aide à la respiration selon la revendication 1 ou 4, caractérisé en ce qu'il comporte une unité de filtrage (**2**) du flux d'air aspiré, l'unité étant disposée en amont de la prise de raccordement (**5**) à l'installation d'oxygénation par rapport au flux d'air aspiré.

7. Dispositif d'aide à la respiration selon la revendication 6, caractérisé en ce que l'unité de filtrage (**2**) comprend un embout d'adaptation (**14**) destiné à être inséré dans le canal (**4**) de l'embout (**1**) et au moins une cartouche de filtration (**16**).

8. Dispositif selon la revendication 7, caractérisé en ce que l'embout d'adaptation (**14**) comporte une zone de transition (**15**) formant un **Venturi**.

9. Dispositif d'aide à la respiration selon la revendication 7 ou 8, caractérisé en ce que l'embout d'adaptation (**14**) est prolongé par deux branches divergentes (**2a**, **2b**) comportant chacune une cartouche de filtration (**16**).

10. Dispositif d'aide à la respiration selon les revendications 8 et 9, caractérisé en ce que la jonction entre les deux branches divergentes et l'embout d'adaptation est constituée de la zone de transition (**15**) dont la section transversale est inférieure à celle des branches divergentes (**2a**, **2b**) pour former le **Venturi**.

## Patentansprüche

1. Vorrichtung zur Sprech- und Atemhilfe für einen tracheotomierten Patienten, wobei die Vorrichtung ein Ansatzstück (1) für die Trachealkanüle (3) enthält, so daß sich ein Kanal (4) ergibt, in welchem ein Sprechventil angeordnet ist, welches durch eine Rückschlagklappe (12), um den Durchfluß ausgeatmeter Luft zu verhindern und den Durchfluß eingeatmeter Luft zu ermöglichen, und eine Stützvorrichtung für besagte Klappe gebildet wird, wobei letztere mittels Fixiervorrichtung (13), die einen Fixierbereich (9) umschreibt, auf einer transversalen Scheidewand montiert ist, welche zur Stützvorrichtung gehört und aus wenigstens zwei Querstreben (8) besteht, welche die Durchgänge für den Luftstrom definieren und welche koaxial zum Ansatzstück in einem gemeinsamen Bereich zusammenlaufen der den Fixierbereich (9) bildet, wobei die besagte Klappe unter Einfluß des geatmeten Luftstroms biegsam gegenüber dem Fixierbereich (9) ist und die Luft hindurchläßt,
dadurch gekennzeichnet, daß
- die Klappe mit einer Stützfläche (11) zusammenwirkt, die zur Stützorrichtung gehört und die durch eine der transversalen Scheidewandflächen gebildet wird, d.h. die zwei Speichen und den gemeinsamen zentralen Bereich, die diese transversale Scheidewand formen,
- die besagte Klappe einerseits aus einem biegsamen und deformierbaren Material hergestellt ist und andererseits nicht vorgespannt ist, so daß sie unter Einfluß der ausgeatmeten Luft gegen die Vorderfläche der Stütze dicht abschließt,
- und daß sie außer dem Sprechventil eine Verbindungsvorrichtung zu einer Fluidquelle zur Unterstützung der Atmung hat, wobei diese Verbindungsvorrichtung ein Verbindungsstutzen (5) ist, der an dem Anatzstück (1) vorgesehen ist und als Verbindung zu einem Saueratoffbeatmungsgerät dient.

2. Vorrichtung nach Anspruch 1, dadurch gekennzeichnet, daß die Stützvorrichtung (7) einen aufgesetzten Rohransatz enthält, der an einer Position im Kanal (4) des Ansatzstückes (1) festgeklemmt ist.

3. Vorrichtung nach Anspruch 1 oder 2, dadurch gekennzeichnet, daß die Fixiereinrichtung (13) eine Befestigung-klemme ist, die die Rückschlagklappe (12) und den gemeinsamen Bereich (9) der Speichen (8) umschließt und somit einen Fixierbereich definiert, der exakt zentriert im Kanal (4) des Ansaugstutzens (1) und auf der durch die Klappe (12) begrenzten Oberfläche ist.

4. Vorrichtung nach Anspruch 1, dadurch gekennzeichnet, daß sie außerdem eine Trennwand (17) enthält, die aufgrund von Öffnungen durch ihre Dicke luftdurchlässig ist und die im Kanal (4) des Ansaugstutzens (1) in bezug auf die ausgeatmete Luft stromabwärts vom Sprechventil eingebaut und befestigt ist.

5. Vorrichtung zur Atemhilfe nach Anspruch 4, dadurch gekennzeichnet, daß die durchlässige Wand (17) durch Speichen gebildet wird.

6. Vorrichtung zur Atemhilfe nach Anspruch 1 oder 4, dadurch gekennzeichnet, daß sie eine Filtereinheit (2) für die Ausatmungsluft enthält, wobei diese Einheit in bezug auf die Ausatmungsluft stromaufwärts vom Verbindungsstutzen (5) zum Sauerstoffbeatmungsgerät angeordnet ist.

7. Vorrichtung zur Atemhilfe nach Anspruch 6, dadurch gekennzeichnet, daß die Filtereinheit (2) ein Übergangsstück (14) enthält, das in den Kanal (4) des Ansatzstückes (1) eingeführt wird und mindestens eine Filterkartusche (16) enthält.

8. Vorrichtung nach Anspruch 7, dadurch gekennzeichnet, daß das Übergangsstück(14) einen Übergangsbereich (15) bildet, der einem Venturirohr entspricht.

9. Vorrichtung zur Atemhilfe nach Anspruch 7 oder 8, dadurch gekennzeichnet daß das Übergangsstück (14) durch zwei divergente Verzweigungsstücke (2a, 2b) verlängert ist, die jeweils eine Filterkartusche (16) enthalten.

10. Vorrichtung zur Atemhilfe nach den Ansprüchen 8 und 9, dadurch gekennzeichnet, daß die Verbindung zwischen den beiden divergenten Verzweigungstücken und dem Übergangsstück als Übergangsbereich (15) ausgedildet ist, dessen transversale Ausdehnung kleiner als die des aus den divergenten Verzweigungsstücken (2a, 2b) gebildeten Venturirohres ist.

## Claims

1. Phonation and respiratory aid device for a tracheotomized patient, device of the type comprising a tip (1) of a tracheotomy cannula (3) defining a channel (4), in which a phonation valve is disposed consisting of a non-return valve (12) for preventing passage of the exhaled air flow and for allowing passage of the inhaled air flow, and of a support means (7) for the said valve, the latter being mounted, by a fixing means (13) defining a fixing zone (9), against a transverse wall belonging to the support means and consisting of at least two crosspieces (8) defining passages for the air flows and joining at a common zone forming the fixing zone (9) coaxial to the tip, the said valve being capable of deforming starting from its fixing zone (9) under the effect of the inhaled air flow and of allowing the latter to pass, characterized in that:
- the valve cooperates with a support face (11) which belongs to the support means and which consists of one of the faces of the transverse wall, that is to say the two crosspieces and the central common zone forming this transverse wall,
- the said valve is, on the one hand, designed and made of a flexible and deformable material and, on the other hand, is not pre-stressed, so that it can be held in sealing support against the support face under the effect of the exhaled air flow,
- and in that it comprises, in addition to the phonation valve, a means for connection to a source of fluid as an element in aiding respiration, this connection means being a connection grip (5) provided in the tip (1) and intended for connection to a forced oxygenation appliance.

2. Device according to Claim 1, characterized in that the support means (7) comprises a bush added and blocked in position in the channel (4) of the tip (1).

3. Device according to Claim 1 or 2, characterized in that the fixing means (13) is a fixing clip tightening on the non-return valve (12) and the common zone (9) of the crosspieces (8), to define the fixing zone which is substantially central to the channel (4) of the tip (1) and to the surface defined by the valve (12).

4. Device according to Claim 1, characterized in that it further comprises a separation wall (17), permeable to the air flow, by means of openings provided in its thickness, interposed and fixed in the channel (4) of the tip (1) downstream of the phonation valve with respect to the inhaled air flow.

5. Respiratory aid device according to Claim 4, characterized in that the permeable wall (17) is constituted by crosspieces.

6. Respiratory aid device according to Claim 1 or 4, characterized in that it comprises a unit (2) for filtering the inhaled air flow, the unit being disposed upstream of the grip (5) for connection to the oxygenation appliance with respect to the inhaled air flow.

7. Respiratory aid device according to Claim 6, characterized in that the filtering unit (2) comprises an adapting tip (14) intended to be inserted in the channel (4 of the tip (1) and at least one filtration cartridge (16).

8. Device according to Claim 7, characterized in that the adapting tip (14) comprises a transition zone (15) forming a Venturi tube.

9. Respiratory aid device according to Claim 7 or 8, characterized in that the adapting tip (14) is extended by two diverging branches (2a, 2b) each comprising a filtration cartridge (16).

10. Respiratory aid device according to Claims 8 and 9, characterized in that the join between the two diverging branches and the adapting tip is constituted by the transition zone (15) of which the cross-section is less than that of the diverging branches (2a, 2b) to form the Venturi tube.
